# NOUVEAU FASCICULE DE BREVET EUROPEEN

(11) **EP 1 671 622 B2**
(45) Date de publication et mention de la décision concernant l'opposition: **10.04.2013**
(45) Mention de la délivrance du brevet: 08.04.2009
(21) Numéro de dépôt: 05301023.7
(22) Date de dépôt: 07.12.2005
(51) Int. Cl.: A61K 8/44, A61K 8/42, A61Q 5/00, A61K 8/46

(54) **Dosage des protéines du cheveu**
Verfahren zur Proteinbestimmung in Haaren
Hair protein assay method

(30) Priorité: 14.12.2004 FR 0452979
(43) Date de publication de la demande: 21.06.2006
(73) Titulaire: L'Oréal, 75008 Paris (FR)
(72) Inventeur: Peron, Marine, 75010 Paris (FR); Lagarde, Arnaud, 76700 Gainneville (FR); Bover, Joël, 95500 Le Thillay (FR)
(74) Mandataire: Le Coupanec, Pascale A.M.P.

(56) Documents cités:
- WO-A2-00/08465
- JP-A- 2001 356 125
- US-A- 5 131 417
- US-B1- 6 730 493
- PATENT ABSTRACTS OF JAPAN vol. 2002, no. 04, 4 août 2002 (2002-08-04) & JP 2001 356125 A (KANEBO LTD), 26 décembre 2001 (2001-12-26)

## Description

La présente invention a pour objet un procédé pour détecter rapidement au niveau de fibres kératiniques, un antécédent de traitement, notamment de type défrisage ou lissage, par technologie alcaline. Elle vise en outre à proposer un kit utile pour la mise en oeuvre d'un tel procédé.

Par "fibres kératiniques", on entend selon l'invention, des fibres d'origine humaine ou animale telles que les cheveux, les poils, les cils, la laine, l'angora, le cachemire ou la fourrure. Bien que l'invention ne soit pas limitée à des fibres kératiniques particulières, il sera néanmoins fait plus particulièrement référence aux cheveux.

Le cheveu est constitué à 85-90% de protéines. Ces protéines appartiennent à deux principales familles : les filaments intermédiaires ou encore kératine et les KAP (Keratin Associated Proteins). Dans son ensemble, la composition protéique du cheveu est riche en cystéine sous sa forme dimère : la cystine.

Les propriétés de mise en forme du cheveu résultent notamment de son architecture protéique.

Ainsi, pour imprimer au cheveu une déformation permanente, on fait généralement appel à des actions chimio-cosmétologiques qui interviennent au niveau des chaînes polypeptidiques des protéines du cheveu. Ces chaînes contiennent des acides aminés soufrés, dont la cystéine sous sa forme dimère (disulfure) : la cystine (environ 15 % en g/100g d'acides aminés totaux). Ces ponts disulfures peuvent être réduits en thiols, puis réoxydés après une déformation imposée afin de rigidifier cette déformation. Cette réaction constitue une des bases cosmétiques des modifications permanentes de la forme de cheveux.

Selon une première technique, on opère par ouverture temporaire du pont disulfure grâce à l'action d'un agent réducteur, en général un sulfite sous forme de sel alcalin ou un thiol tel que l'acide thioglycolique. Cette action de rupture a comme effet de casser les ponts disulfures, assurant ainsi une plasticité et une capacité de mouvement aux chaînes polypeptidiques les unes par rapport aux autres. Les ponts sont ensuite reformés en d'autres points après la déformation désirée, par fixation avec un agent oxydant.

Une seconde technique consiste à effectuer une opération dite de lanthionisation, à l'aide d'une composition contenant une base appartenant à la famille des hydroxydes. Elle conduit à remplacer une partie des cystines par des lanthionines (liaisons monosulfures -CH₂-S-CH₂). Cette opération de lanthionisation fait intervenir dans un premier temps une réaction de β-élimination sur la cystine, à l'aide d'un ion hydroxyde, puis à faire réagir la déhydroalanine ainsi obtenue avec un groupe thiol. Par rapport à la première technique, mettant en oeuvre un agent réducteur, cette technique de lanthionisation ne nécessite pas d'étape de fixation. La formation des ponts lanthionine est irréversible. Cette technique permet donc d'obtenir en une seule étape le défrisage ou le lissage des fibres kératiniques.

En conséquence, les deux techniques précitées, quoique conduisant au même résultat, à savoir une déformabilité permanente, affectent de manière différente la structure du cheveu. En particulier, le professionnel du domaine capillaire sait que la causticité des dérivés alcalins mis en oeuvre selon la seconde technique est susceptible, dans certains cas, d'affecter significativement l'état du cheveu en le rendant notamment beaucoup plus fragile. Cette fragilité s'accompagne d'une perte de substances et peut aller jusqu'à la rupture des cheveux notamment s'ils sont exposés à des traitements consécutifs de ce type.

Il existe déjà des méthodes pour caractériser un antécédent de défrisage alcalin, notamment par la détection de la présence de lanthionine au sein des fibres kératiniques. Cette détection s'effectue soit par un dosage de la lanthionine après hydrolyse acide et analyse des acides aminés, soit par une analyse en spectroscopie confocale RAMAN.

De même, plusieurs méthodes de collecte des protéines ont déjà été proposées. Ainsi, pour extraire les protéines corticales, on sait qu'il est possible de détendre les protéines du cheveu dans une solution aqueuse d'urée, puis de couper les ponts disulfures par le 2-mercaptoéthanol ou l'acide thioglycolique (MacLaren. J.A. & Kilpatrick D.J., Aust. J. Biol. Sci., 21, 805-813 [1968]). Plus récemment, le document JP 2002-114798 A a proposé de combiner des composés uréiques particuliers en présence d'un réducteur. La méthode de collecte plus particulièrement décrite vise à éluer et collecter les protéines kératiniques des microfibrilles et de la matrice constituant la partie corticale du cheveu en traitant ceux-ci par un mélange d'urée et de thiourée, dans un rapport compris entre 5/1 et 1/2 et à collecter à partir du résidu d'élution, la partie cuticulaire maintenue dans sa forme.

Il est clair que l'ensemble de ces techniques, bien que très fiables en terme de diagnostic, s'avère très contraignant en termes de mise en oeuvre et donc coûteux.

Il demeure donc aujourd'hui un besoin pour le professionnel de disposer d'un moyen fiable, rapide et ne nécessitant pas de matériel scientifique élaboré pour identifier l'"historique" d'un cheveu de manière à apprécier au mieux la compatibilité de traitement(s) consécutif(s) avec son état, notamment protéique.

Au sens de l'invention, l'état protéique de fibres kératiniques entend désigner leur concentration protéique selon une échelle qualificative et non quantitative, en d'autres termes, leur richesse protéique.

De manière inattendue, les inventeurs ont constaté qu'un test basé sur la solubilisation des protéines kératiniques mais ne requièrant en revanche aucunement l'isolement consécutif de celles-ci en vue de leur caractérisation, permettait de discriminer efficacement un antécédent de défrisage par technologie alcaline des autres traitements, par exemple de types coloration et/ou défrisage thioglycolique.

La présente invention vise précisément à proposer un procédé utile et rapide pour détecter si une fibre kératinique a déjà été exposée à un traitement alcalin de type défrisage par technologie alcaline, quelque soit le degré de sensibilisation de cette fibre kératinique.

De manière usuelle, un cheveu qualifié de "sensibilisé" est un cheveu ayant subi au moins un traitement d'oxydation par exemple lors d'une coloration ou décoloration par opposition à un cheveu naturel.

Plus précisément, la présente invention vise, selon un premier aspect, un procédé pour détecter au niveau de fibre(s) kératinique(s), un antécédent de traitement par technologie alcaline, notamment de type défrisage ou lissage, comprenant au moins la mise en contact desdites fibres kératiniques avec une solution dite d'extraction contenant au moins de l'urée, de la thiourée ou un de leurs dérivés en mélange avec au moins un réducteur autre que la thiourée, dans des conditions efficaces pour solubiliser des protéines kératiniques, le dosage qualitatif et/ou quantitatif des protéines au sein de ladite solution d'extraction, et la caractérisation d'un antécédent de traitement alcalin de défrisae ou de lissage par la détection d'une quantité très réduite, voire nulle, de protéines sous forme solubilisée.

La présente invention vise, selon un autre de ses aspects, un kit utile pour caractériser l'état protéique de fibre(s) kératinique(s), et notamment détecter, au niveau des fibre(s) kératinique(s), un antécédent de traitement par technologie alcaline, ledit kit comportant au moins
- une solution contenant de l'urée et de la thiourée ou un de leurs dérivés,
- au moins un réducteur autre que la thiourée,
- au moins un composé utile à titre d'indicateur colorimétrique pour doser qualitativement et/ou quantitativement des protéines kératiniques.

Au sens de l'invention, l'expression "dosage qualitatif des protéines" désigne un dosage visant à caractériser l'abondance relative des protéines sans évaluer précisément leur concentration. En d'autres termes, le dosage permet à son utilisateur d'estimer la richesse en protéines de la solution d'extraction.

Un dosage quantitatif vise, en revanche, à déterminer la concentration précise de la solution d'extraction en protéines, comme illustré dans les exemples présentés ci-après.

Avantageusement, le procédé selon l'invention permet de caractériser l'existence d'un antécédent de traitement par défrisage alcalin, quelque soit le ou les traitement(s) de sensibilisation subi(s) par ailleurs antérieurement et/ou postérieurement par le cheveu considéré. Le procédé selon l'invention s'avère efficace sur toute nature de cheveux, à savoir naturels ou sensibilisés.

### Solution d'extraction des protéines kératiniques

Comme précisé précédemment, le mélange mis en oeuvre pour l'extraction des protéines est une solution associant au moins de l'urée et de la thiourée ou un de leurs dérivés et un agent réducteur autre que la thiourée.

Au sens de l'invention, les dérivés de thiourée ou urée se réfèrent à leurs dérivés de type N-alkylés et N-hydroxyalkylés. Ces motifs alkylés peuvent être en C₁ à C₁₀ et de préférence en C₁ à C₄ et, le cas échéant, être substitués, sous réserve que ces substituants ne soient pas susceptibles d'interférer sur la réactivité de l'urée ou thiourée.

La solution destinée à réagir vis-à-vis des protéines kératiniques comprend généralement un mélange d'urée et de thiourée ou d'un de leurs dérivés dans un rapport urée/thiourée variant de 5/1 à 1/2 en particulier de 4/1 à 2/1.

Plus particulièrement, un tel mélange est généralement présent à raison de 20 à 80 % en poids, en particulier de 30 à 70 % en poids par rapport au poids total de la solution d'extraction.

Ces deux composés sont associés à une quantité efficace d'au moins un réducteur autre que la thiourée.

Au sens de l'invention, un réducteur désigne un composé susceptible de couper les ponts disulfures dans les fibres kératiniques et, plus particulièrement, en vue de les réduire.

Conviennent ainsi, notamment, les dérivés de type thiol, phosphine et sulfite.

Selon un mode de réalisation particulier de l'invention, il s'agit d'un agent réducteur de type thiol.

De tels agents sont notamment choisis parmi le dithiothréitol, l'acide thioglycolique ou l'acide thiolactique et leurs dérivés esters et amides, notamment le monothioglycolate de glycérol, la cystéamine et ses dérivés acylés en C₁-C₄ tels que la N-acétyl-cystéamine ou la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides tels que ceux décrits dans la demande de brevet EP-A-354 835, les N-mono ou N,N-di-alkyl-mercapto-4-butyramides tels que ceux décrits dans la demande de brevet EP-A-368 763, les aminomercaptoalkylamides, tels que ceux décrits dans la demande de brevet EP-A-432 000, les dérivés des acides N-(mercaptoalkyl)succinamiques et des N-(mercaptoalkyl)succinimides tels que ceux décrits dans la demande de brevet EP-A-465 342, les alkylamino mercaptoalkylamides tels que ceux décrits dans la demande de brevet EP-A-514 282, le mélange azéotrope de thioglycolate de 2-hydroxypropyle et de thioglycolate de (2-hydroxy-1-méthyl)éthyle tels que ceux décrits dans la demande de brevet FR-A-2 679 448, les mercaptoalkylaminoamides tels que ceux décrits dans la demande de brevet FR-A-2 692 481, les N-mercaptoalkylalcanediamides tels que ceux décrits dans la demande de brevet EP-A-653 202, ainsi que les dérivés d'acide formamidine sulfinique tels que ceux décrits dans la demande PCT/LTS01/43124, déposée par la Demanderesse.

Ce réducteur est bien entendu utilisé à une concentration suffisante pour obtenir l'effet recherché, à savoir la réduction des ponts disulfures. Pour des raisons évidentes, cette quantité est susceptible de varier significativement selon la nature du réducteur considéré.

En particulier, cette concentration peut être de l'ordre de 0,01 à 2 M, en particulier de 0,03 à 0,5 M et plus particulièrement de 0,04 à 0,1 M.

Conviennent plus particulièrement à titre d'agent réducteur le 2-mercaptoéthanol, le dithiothreitol (DTT), l'acide thioglycolique, l'un de leurs dérivés ou leurs mélanges.

Il est également possible d'envisager d'associer à ce réducteur, un ou plusieurs composés connus pour leur aptitude à renforcer son efficacité. A titre illustratif, de tels composés peuvent se présenter sous la forme d'un mélange SIO₂/PDMS (polydiméthylsiloxane), de diméthylisosorbitol, pyrrolidone, N-alkylpyrrolidone, thiamorpholinone, d'alkyléthers d'alkylèneglycol, de dialkylèneglycol, d'alcanes diols en C₃-C₆ ou de leurs mélanges.

De tels composés peuvent être présents à raison de 0,001 à 10 % en poids par rapport au poids de la composition.

Outre l'urée, la thiourée et le composé réducteur, la solution destinée à solubiliser les protéines kératiniques peut également contenir un agent tensioactif de type non ionique, anionique, cationique ou amphotère. Il peut notamment s'agir des alkylsulfates, des alkylbenzènesulfonates, des alkyléthersulfates, des alkylsulfonates, des alkylbétaïnes, des alkylphénols oxyalkylénés, des alcanolamides d'acides gras, des esters d'acides gras oxyalkylénés, ainsi que des alcools gras oxyalkylénés et des alkylpolyglucosides.

La solution d'extraction peut également comprendre un ou plusieurs agents acidifiants ou basifiants et en particulier le TRIS (TRIS[hydroxyméthyl]aminométhane).

Bien entendu, ces agents sont choisis de manière à ne pas porter préjudice à la réactivité recherchée vis-à-vis des protéines kératiniques.

Comme précisé précédemment, le procédé selon l'invention implique la mise en contact de fibres kératiniques avec une solution d'extraction conforme à l'invention.

Généralement, un échantillon de type méchette correspondant à environ une dizaine de cheveux est suffisant pour la réalisation du procédé conforme à l'invention.

Plus précisément, le rapport pondéral entre les fibres kératiniques à analyser et la solution d'extraction varie de 0,2 à 100 mg/ml, notamment de 1 à 50 mg/ml et plus particulièrement est de l'ordre de 10 mg/ml.

La quantité de fibres kératiniques est généralement de l'ordre de 50 mg. Quant au volume de la solution d'extraction, il est généralement de l'ordre de 5 ml.

La méchette de fibres kératiniques est mise en contact avec la solution d'extraction, généralement par immersion directement dans un récipient, de type tube ou cuve par exemple, contenant ladite solution. Ces fibres kératiniques peuvent avantageusement être découpées au préalable en tronçons de tailles réduites à l'échelle de 2 à 3 mm par exemple.

Cette méchette est généralement prélevée par coupe au niveau de la chevelure dont on cherche à caractériser un antécédent de traitement de défrisage par technologie alcaline.

Les inventeurs ont mis en évidence que la réactivité de la solution d'extraction est significativement accrue si l'on conduit la réaction de solubilisation des protéines à chaud, c'est-à-dire à une température supérieure à 50 °C.

En conséquence, pour des raisons de rapidité, la solution contenant la méchette est avantageusement chauffée à une température supérieure à 50 °C, plus particulièrement supérieure à 70 °C et notamment supérieure ou égale à 100 °C. Il est entendu que cette température doit être compatible avec le cheveu et ne doit pas le détruire.

Ce chauffage peut être appliqué par toute technique conventionnelle. Il peut ainsi s'agir d'un chauffage de type bain-marie, chauffe-biberon, micro-ondes et/ou sèche-cheveux.

Bien entendu, le temps nécessaire à l'extraction peut varier significativement de la minute, voire la seconde, à plusieurs heures selon les conditions retenues, notamment en terme de température, pour sa réalisation.

D'une manière générale, l'extraction des protéines est obtenue dans un délai de l'ordre de 15 minutes, lorsque la réaction est effectuée à 100 °C, contre un délai de 18 heures pour une réaction réalisée à 40 °C.

Ce chauffage peut par ailleurs être effectué avec agitation concomitante du récipient contenant les fibres kératiniques et la solution d'extraction.

La mesure ou évaluation de l'abondance relative des protéines solubilisées au sein de la solution d'extraction peut bien entendu être réalisée à l'aide de différentes méthodes.

On peut ainsi envisager de caractériser l'abondance en protéines au sein de la solution, par une méthode de dosage de type physique, comme par exemple diffusion de la lumière turbidité, de type chimique, physico-chimique ou encore de type immunologique.

Toutefois, dans la mesure où ce test est plus particulièrement destiné à des professionnels du coiffage, il est avantageux d'associer à cette méthode d'extraction, un mode de détection par colorimétrie, notamment basé sur la méthode de Bradford ou Lowry. De tels tests sont couramment utilisés pour le dosage des protéines.

Selon cette alternative, on peut mettre en présence tout ou partie de la solution de traitement, susceptible de contenir des protéines sous une forme solubilisée, avec une quantité efficace d'un composé, encore dénommé ci-après "indicateur colorimétrique", susceptible d'interagir avec celles-ci et de manifester cette interaction selon un mode colorimétrique. Il peut s'agir soit d'une apparition de couleur soit de la modification, voire disparition de sa couleur initiale.

Cette mise en contact de la solution d'extraction avec un indicateur colorimétrique peut en particulier être réalisée en introduisant un échantillon déterminé de ladite solution dans une solution contenant l'indicateur colorimétrique. La mise en contact de l'indicateur colorimétrique avec tout ou partie de la solution d'extraction peut requérir au préalable la dilution de cette solution d'extraction. Cette option est bien entendu fonction de la nature de l'indicateur colorimétrique et est généralement prescrite dans le protocole d'utilisation de l'indicateur colorimétrique.

On peut également envisager une variante de réalisation selon laquelle l'indicateur colorimétrique est fixé, notamment par adsorption, sur un support solide par exemple de type bandelette à l'image par exemple du papier pH. La mise en contact se fait alors par imprégnation du support avec tout ou partie de la solution d'extraction.

L'intensité de la couleur obtenue à l'issue de la mise en contact a en outre pour avantage de pouvoir être parallèlement un indicateur de la concentration en protéines, sous réserve de disposer, avec l'indicateur coloré retenu, d'un étalonnage correspondant.

Selon un mode de réalisation particulier de l'invention, l'indicateur est plus à base d'une solution acide de bleu de Coomassie G-250, susceptible d'évoluer en terme de nuances colorées dans une gamme de coloration variant de 465 µm à 585 µm, lorsqu'il est associé à des protéines.

A titre illustratif d'un test colorimétrique commercial convenant à l'invention, on peut particulièrement cité celui distribué par la société BIO RAD sous le nom de BIO RAD PROTEIN ASSAY®, kit commercial de dosage de protéines basé sur la méthode de Bradford.

Pour les raisons évoquées précédemment, ce mode de détection de type colorimétrique est particulièrement intéressant en terme de facilité de manipulation et de rapidité de lecture.

Il peut en outre être souhaitable de disposer parallèlement d'un témoin colorimétrique permettant de qualifier l'abondance relative en protéines. Ce témoin peut être figuré par un mélange de protéines kératiniques de concentration déterminée et sous une forme purifiée, notamment extraites des cheveux naturels. Toutefois, en absence d'une telle référence, il est possible d'utiliser n'importe quelle autre protéine à titre de standard relatif. Pour des raisons évidentes, il est avantageux de privilégier le choix d'une protéine de référence qui donnera une intensité de couleur proche de celle de la protéine à doser.

Conviennent ainsi notamment, comme protéines standard, les protéines de type sérum albumine bovine ou γ-albumine bovine qui sont des protéines disponibles commercialement sous des formes purifiées.

Comme il ressort des exemples figurant ci-après, la détection d'un antécédent de traitement alcalin de défrisage ou de lissage se caractérise par la présence d'une quantité très réduite, voire nulle de protéines sous forme solubilisée. En revanche, en absence d'un tel antécédent, le procédé conforme à l'invention permet de caractériser, voire de quantifier, la présence d'une quantité significative en protéines kératiniques au sein de la solution d'extraction. Cette présence peut se traduire, dans le cas d'un mode de détection par colorimétrie, par la manifestation d'une couleur dont l'intensité est proportionnelle à la quantité en protéines solubilisées.

L'invention concerne également un kit utile pour caractériser l'état protéique de fibres kératiniques, et notamment utile pour caractériser un antécédent de traitement par technologie alcaline de défrisage ou de lissage, comportant au moins:
- une solution comprenant au moins de l'urée et de la thiourée ou un de leurs dérivés
- un agent réducteur autre que la thiourée, et
- au moins un composé utile à titre d'indicateur colorimétrique pour doser qualitativement et/ou quantitativement les protéines kératiniques extraites.

L'ensemble des composants constituant la solution destinée à l'extraction des protéines peut être conditionné selon une première variante dans un même récipient, ou selon une seconde variante dans des récipients séparés.

Selon cette seconde alternative, la solution de thiourée et d'urée d'une part, et le réducteur d'autre part, sont conditionnés de manière séparée. Dans ce cas, les récipients qui leur sont respectivement dédiés peuvent être conformés de manière à être adaptés à un mélange de l'ensemble de leurs composants préalablement à la réalisation du test. Par exemple, on peut envisager que ces deux récipients soient réunis de manière à former un unique récipient au moment de la réalisation du test.

Selon un premier mode de réalisation, le récipient contenant la solution à base d'urée, de thiourée et, le cas échéant, l'agent réducteur est conformé pour être adapté à l'immersion de fibres kératiniques. Dans ce cas, il peut être muni d'un obturateur de type capuchon amovible destiné à être ôté au moment de la réalisation du test.

Une autre variante peut impliquer l'introduction et la mise en contact de l'échantillon de fibres kératiniques à caractériser, non pas dans le mélange préalablement constitué des deux composants de la solution d'extraction, mais avec seulement l'un des composants à savoir la solution comprenant au moins l'urée et la thiourée ou l'agent réducteur. Selon cette alternative, le second composant est associé consécutivement au mélange préliminaire des fibres avec le premier composant.

Selon un autre mode de réalisation, le kit peut contenir en outre un récipient distinct destiné à recevoir la solution d'urée, et de thiourée, l'agent réducteur et les fibres kératiniques dans un ordre non déterminé.

Quelque soit le mode de réalisation, le récipient dédié à l'immersion de la méchette pendant le déroulement du test est compatible avec un chauffage à une température au moins égale à 50 °C, de préférence supérieure à 70 °C, voire de l'ordre de 100 °C. Il peut par exemple être conformé pour un chauffage de type chauffe-biberon.

En ce qui concerne l'indicateur colorimétrique, il est conditionné généralement en solution dans un récipient qui lui est spécifique.

L'ensemble des récipients constituant le kit sont obturés lors de leur conditionnement. Les modes d'obturation sont ajustés de manière à être propices à une ouverture aisée au moment de la réalisation du test et, le cas échéant, à une libération contrôlée de leur contenu par l'adaptation d'un embout compte-goutte par exemple. Cette dernière option est notamment intéressante pour l'étape de détection par colorimétrie.

Le kit selon l'invention comprend en outre avantageusement une notice d'utilisation dictant à l'utilisateur les consignes à suivre pour la réalisation du test.

D'une manière générale, le protocole à suivre peut impliquer les étapes suivantes :
- la mise en présence de fibres kératiniques, plus particulièrement d'une méchette de cheveux, généralement prédécoupées à l'état de tronçons de 2 à 3 mm, avec la solution d'urée et de thiourée et l'agent réducteur, ces deux composants de la solution d'extraction ayant été au préalable mélangés ou étant mélangés en présence desdites fibres kératiniques,
- si nécessaire, l'exposition du récipient contenant le mélange précédent à une source de chaleur, le cas échéant sous agitation, de manière à porter la solution d'extraction à une température supérieure à 50 °C voire 100 °C pendant un délai déterminé, par exemple environ 15 minutes pour un chauffage à 100 °C,
- si nécessaire, la dilution de la solution d'extraction, à l'issue de cette étape de chauffage,
- l'introduction d'une quantité donnée de cette solution dans le récipient contenant l'indicateur colorimétrique ou inversement d'une quantité donnée de l'indicateur colorimétrique dans ladite solution d'extraction, et
- la lecture du résultat en se référant, le cas échéant, à un témoin colorimétrique.

Ce témoin colorimétrique peut être fourni au sein du kit voire présenté au niveau du conditionnement du kit.

On peut également envisager de fournir avec le kit un nuancier permettant de doser qualitativement et/ou quantitativement les protéines extraites en fonction de l'intensité de couleur obtenue à l'issue du test.

Le kit selon l'invention peut également contenir des fibres kératiniques de référence pour guider l'utilisateur sur l'importance de l'échantillon à prélever au niveau des fibres kératiniques, et notamment de la chevelure à tester.

L'invention pourra être mieux comprise à la lecture de la description détaillée qui va suivre, d'exemples de mises en oeuvre non limitatifs de celle-ci et à l'examen du dessin annexé sur lequel :
- la figure 1 représente de manière schématique, en perspective, un dispositif de conditionnement d'un kit conforme à un exemple de mise en oeuvre de l'invention,
- la figure 2 représente un exemple de kit conforme à l'invention,
- la figure 3 représente de manière schématique un dispositif de chauffage pouvant être utilisé pour porter la méchette et la solution d'extraction à une température prédéfinie, et
- la figure 4 est une vue schématique en coupe axiale d'une variante de réalisation d'un dispositif de conditionnement permettant de réaliser le mélange extemporané de la solution d'extraction et du réducteur.

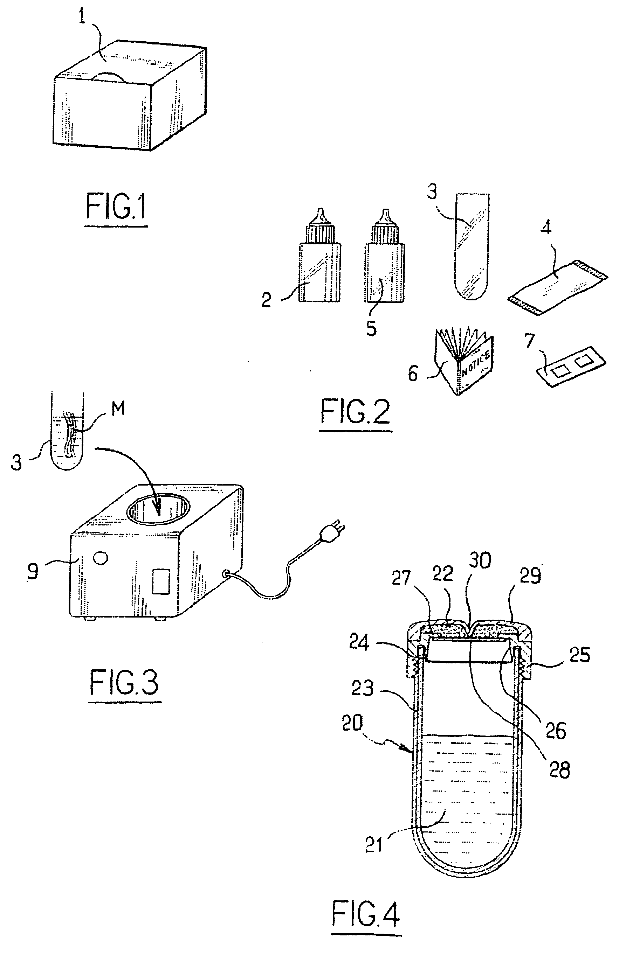

Un kit permettant de caractériser un antécédent de traitement capillaire peut être proposé, par exemple à l'utilisateur, dans une boite 1 contenant, comme on peut le voir sur la figure 2, un récipient 2 contenant une solution d'urée et de thiourée, un tube 3 ou autre récipient destiné à recevoir la méchette et la solution d'extraction, un sachet 4 ou autre dispositif de conditionnement, comprenant le réducteur, un récipient 5 contenant l'indicateur colorimétrique, une notice d'utilisation éventuelle 6 et une échelle colorimétrique 7 permettant à l'utilisateur d'apprécier la couleur finale du mélange en fin de réaction.

Le kit peut servir à un usage unique ou au contraire comporter des quantités suffisantes de réactifs pour permettre la caractérisation de plusieurs méchettes.

Dans une variante non illustrée, la notice 6 est remplacée par des indications figurant sur la boite 1. Il peut en être de même de l'échelle colorimétrique 7, laquelle peut figurer sur la notice ou sur la boite 1, voire sur l'un des récipients 4 ou 5, le cas échéant.

Dans l'exemple de la figure 2, la solution d'extraction contenue dans le récipient 2 contient déjà un mélange d'urée et de thiourée, mais on ne sort pas du cadre de la présente invention lorsque la solution d'extraction est préparée extemporanément par mélange d'urée et de thiourée.

Pour porter la solution d'extraction avec une méchette M dans le tube 3 à une température prédéfinie, on peut utiliser un appareil de chauffage 9, représenté schématiquement à la figure 3.

On a représenté à la figure 4 un exemple de dispositif de conditionnement 20 agencé pour stocker séparément la solution urée et thiourée 21 et le réducteur 22 tout en permettant leur mélange ultérieur.

Le dispositif 20 comporte par exemple un corps 23 et une partie amovible 24 agencée pour se fixer sur le corps 23, par exemple par vissage ou encliquetage. La partie amovible 24 comporte une base 25 pourvue d'une lèvre annulaire d'étanchéité 26 s'appliquant sur le corps 23 et d'un rebord 27 servant de support à un opercule 28 permettant d'isoler le réducteur 22 de la solution urée et thiourée 21 durant le stockage.

La partie amovible 24 comporte également une partie supérieure 29 sur laquelle l'utilisateur peut appuyer pour briser l'opercule 28 et provoquer l'expulsion du réducteur 22 dans le corps 23 et son mélange avec la solution urée et thiourée 21.

La partie supérieure 29 comporte, dans l'exemple illustré, dans sa portion centrale, un relief 30 de préférence de forme pointue permettant de perforer l'opercule 28. Ce dernier est par exemple constitué par un film thermosoudé à sa périphérie sur le rebord 27. La partie supérieure 29 est par exemple fixée par encliquetage ou thermosoudage sur la partie de base 25, après mise en place du réducteur 22.

Pour utiliser le dispositif représenté à la figure 4, l'utilisateur peut retirer la partie amovible 24 et introduire la méchette dans le corps 23, de façon à l'exposer à la solution d'extraction 21. Le corps 23 peut être disposé dans un appareil de chauffage comme illustré à la figure 3. Une fois l'opération d'extraction terminée, l'utilisateur peut remettre en place la partie amovible 24 sur le corps 23 et appuyer sur la partie supérieure 29 pour provoquer la rupture de l'opercule 28 et l'adjonction du réducteur 22 dans la solution d'extraction 21.

Bien entendu, d'autres dispositifs de conditionnement peuvent encore être utilisés.

### EXEMPLES

L'ensemble des essais décrits ci-après a été effectué en utilisant les fibres kératiniques suivantes :
(A des fins de clarté, les sigles de codages suivants ont été adoptés, N : naturel, C : coloré, D : décoloré, TA : Traitement défrisage alcalin, TT : traitement défrisage thiolé).

### CHEVEU CAUCASIEN NON TRAITE PAR DEFRISANT:

N : Cheveux Naturels
D : Cheveux Décolorés
C: Cheveux Colorés

### CHEVEU CAUCASIEN TRAITE PAR DEFRISANT :

### TECHNOLOGIE ALCALINE (TA) :

TAG : Traitement Alcalin de défrisage à base de carbonate de Guanidine.
TAS : Traitement Alcalin de défrisage à base de Soude

### TECHNOLOGIE THIOLEE (TT):

TT = Traitement Thiolé de défrisage à base d'acide thioglycolique

### SOLUTION D'EXTRACTION

La solution réductrice utilisée à raison d'un volume de 5 ml par essai est préparée à partir des composés suivants aux concentrations précises :
- Urée (7M) commercialisée par BIORAD référence : 1610730.
- Thiourée (2M) commercialisée par FLUKA référence : 88810,
- DTT (Dithiothreitol) (0,05M) commercialisé par SIGMA référence : D-9779,
- Tris (0,05M) commercialisé par ALDRICH référence : T8,760-2
- Triton X (0,1 %) commercialisée par SIGMA référence : T-8787,
- Eau Milli-Q.

### METHODE DE DETECTION

En ce qui concerne la méthode de détection utilisée pour caractériser les protéines solubilisées, il s'agit d'une méthode colorimétrique reposant sur la méthode Bradford (Bradford, Anal Biochem 72, 248, 1976).

Plus particulièrement, le test utilisé est le test Bio-Rad Protein® commercialisé par la société BIO RAD et qui utilise le bleu de Coomassie® à titre d'indicateur colorimétrique.

La protéine d'étalonnage utilisée est celle conseillée par le test à savoir la protéine de sérum albumine bovin (BSA) commercialisée par CALBIOCHEM sous la référence commerciale 12659.

### EXEMPLE 1

Le protocole d'extraction suivi pour l'ensemble des essais est le suivant:

Une méchette de fibres capillaires (correspondant à environ 50 mg) est coupée en tronçons de 2 à 3 mm avec des ciseaux qui sont mis en contact avec 5 ml de la solution d'extraction décrite précédemment. Cette mise en contact est effectuée soit à 40°C pendant 18 h soit à une température de 100 °C procurée par chauffage au bain-marie du milieu d'extraction pendant 15 minutes. Le tout est maintenu sous agitation à l'aide d'un barreau aimanté tout au long de l'extraction.

A l'issue de l'extraction, les protéines dans le surnageant sont dosées à l'aide du kit colorimétrique commercial Bio Rad Protein Assay®.

Pour ce faire, on procède à l'ajout de 30 µl du surnageant à 5 ml du réactif colorimétrique de Bradford (préalablement dilué au 1/5^{ème} et filtré selon les consignes précisées dans le kit de BIO RAD). On laisse la couleur se développer quelques minutes.

La lecture des résultats est comme suit :
- si une couleur se développe (allant du marron vers le bleu), le cheveu n'a pas subi de défrisage alcalin,
- si la couleur ne change pas ou très peu, le cheveu a subi un défrisage alcalin.

A partir d'un nuancier de couleur préalablement établi en fonction de concentrations de protéines obtenues avec divers traitements préalablement subis par les cheveux, il est possible de lire le résultat visuellement et répondre à la question de l'antécédent ou non d'un défrisage alcalin pour le cheveu testé.

A partir d'une gamme étalon de protéines, il est possible de quantifier les protéines dans les solutions d'extraction et de calculer un taux d'extraction rapportée à la masse de cheveu utilisée. Les résultats figurent dans les tableaux ci-après. Les résultats y sont exprimés en g de protéines extraites pour 100 g de cheveux secs et en se referant à la BSA.

Les résultats obtenus sont présentés dans les tableaux I à III ci-après.

**TABLEAU I**

| **Cheveux non traités par un défrisant** | | 18h | 15 min |
|---|---|---|---|
| | | 40 °C | 100 °C |
| | | Extrait % | Extrait % |
| CN | Cheveux naturels - témoin | 13,7 | 16,0 |
| CC | Cheveux colorés | 16,6 | 15,5 |
| CD | Cheveux décolorés | 20,8 | 15,9 |

**TABLEAU II**

| **Cheveux défrisés avec une technologie thiolée** | | 18h | 15 min |
|---|---|---|---|
| | | 40 °C | 100 °C |
| | | Extrait % | Extrait % |
| CN + TT | Cheveux naturels - défrisés Ac. thioglycolique | 11,0 | 12,3 |
| CC + TT | Cheveux colorés défrisés Ac. thioglycolique | 15,2 | 12,3 |
| CD + TT | Cheveux décolorés défrisés Ac. thioglycolique | 15,6 | 15,0 |

**TABLEAU III**

| **Cheveux défrisés avec une technologie alcaline** | | 18h | 15 min |
|---|---|---|---|
| | | 40 °C | 100 °C |
| | | Extrait % | Extrait % |
| CN + TAG | Cheveux naturels + défrisés carbonate de guanidine | 0,0 | 0,2 |
| CC + TAG | Cheveux colorés + défrisés carbonate de guanidine | 0,6 | 0,6 |
| CD + TAG | Cheveux décolorés + défrisés carbonate de guanidine | 3,0 | 2,6 |
| CN + TAS | Cheveux naturels + défrisés soude | 0,4 | 0,5 |
| CC + TAS | Cheveux cololrés + défrisés soude | 0,7 | 1,2 |
| CD + TAS | Cheveux décolorés + défrisés soude | 4,4 | 3,1 |

On note que les cheveux préalablement traités par une technologie alcaline se repèrent immédiatement par le manque de développement de la couleur (restent marron ou deviennent légèrement verts) lors du test colorimétrique, ce qui se traduit par une extraction très faible de protéines alors que les fibres kératiniques n'ayant pas subi de traitement de défrisage alcalin développent une couleur bleue associée à des taux d'extraction des protéines beaucoup plus élevés.

### EXEMPLE 2

Les essais précédents ont été répétés sur des méchettes de cheveux directement prélevés sur la tête. Les résultats sont présentés dans le tableau IV ci-après :

**TABLEAU IV**

| Codage | Traitement | Extrait % |
|---|---|---|
| A | Lissage Thiolé | 11,4 |
| B | Défrisage carbonate de guanidine | 0,3 |
| C | Lissage Thiolé | 18,5 |
| D | Lissage Thiolé | 14,1 |
| E | Défrisage carbonate de guanidine | 0,3 |
| F | Lissage Thiolé | 20,6 |

Les résultats précédents sont confirmés.

## Revendications

1. Procédé pour détecter, au niveau de fibre(s) kératinique(s), un antécédent de traitement par technologie alcaline de défrisage ou de lissage comprenant au moins la mise en contact desdites fibre(s) kératinique(s) avec une solution dite d'extraction comprenant au moins de l'urée et, de la thiourée ou un de leurs dérivés, en mélange avec au moins un réducteur autre que la thiourée, dans des conditions efficaces pour solubiliser des protéines kératiniques, le dosage qualitatif et/ou quantitatif des protéines de ladite solution d'extraction et la caractérisation d'un antécédent de traitement alcalin de défrisage ou de lissage par la détection d'une quantité très réduite, voire nulle, de protéines sous forme solubilisée.

2. Procédé selon la revendication 1, **caractérisé en ce qu'**il comprend le dosage qualificatif des protéines solubilisées dans la solution d'extraction.

3. Procédé selon la revendication 1 ou 2, **caractérisé en ce que** la solution d'extraction comprend un mélange d'urée et de thiourée dans un rapport urée/thiourée variant de 5/1 à 1/2 en particulier de 4/1 à 2/1.

4. Procédé selon la revendication 3, **caractérisé en ce que** ledit mélange est présent à raison de 20 à 80 % en poids, en particulier de 30 à 70 % en poids par rapport au poids total de ladite solution d'extraction.

5. Procédé selon l'une quelconque des revendications précédentes, **caractérisé en ce que** ladite solution comprend au moins un réducteur de type thiol.

6. Procédé selon la revendication 5, **caractérisé en ce que** ledit réducteur est choisi parmi l'acide thioglycolique ou l'acide thiolactique et leurs dérivés esters et amides, notamment le monothioglycolate de glycérol, la cystéamine et ses dérivés acylés en C₁-C₄ tels que la N-acétyl-cystéamine ou la N-propionyl-cystéamine, la cystéine, la N-acétyl-cystéine, l'acide thiomalique, la panthétéine, l'acide 2,3-dimercaptosuccinique, les sulfites ou bisulfites d'un métal alcalin ou alcalino-terreux, les N-(mercaptoalkyl)-ω-hydroxyalkylamides, les N-mono- ou N,N-di-alkylmercapto-4-butyramides, les aminomercaptoalkylamides, les dérivés des acides N-(mercaptoalkyl)succinamiques et des N-(mercaptoalkyl)succinimides, les akylaminomercaptoalkylamides, le mélange azéotrope de thioglycolate de 2-hydroxypropyle et de thioglycolate de (2-hydroxy-1-méthyl)éthyle, les mercaptoalkylaminoamides, les N-mercaptoalkylalcanediamides, et les dérivés d'acide formamidine sulfinique

7. Procédé selon la revendication 6, **caractérisée en ce que** ledit réducteur est choisi parmi le 2-mercaptoéthanol, le dithiothreitol (DTT), l'acide thioglycolique, l'un de leurs dérivés ou de leurs mélanges.

8. Procédé selon l'une quelconque des revendications précédentes, **caractérisé en ce que** ledit réducteur est présent en raison de 0,01 à 2 M, en particulier de 0,03 à 0,5 M et plus particulièrement de 0,04 à 0,1 M dans la solution d'extraction.

9. Procédé selon l'une quelconque des revendications précédentes, **caractérisé en ce que** les fibres kératiniques et la solution d'extraction sont mises en contact à raison de 0,2 à 100 mg, et plus particulièrement à raison de 1 à 50 mg et notamment à raison d'environ 10 mg de fibres kératiniques de solution d'extraction.

10. Procédé selon l'une quelconque des revendications précédentes, **caractérisé en ce que** les fibres kératiniques sont immergées dans la solution d'extraction et l'ensemble est chauffé à une température supérieure à 50 °C, plus particulièrement supérieure à 70 °C et notamment supérieure ou égale à 100 °C.

11. Procédé selon l'une quelconque des revendications précédentes, **caractérisé en ce que** le dosage des protéines est réalisé par une méthode de dosage chimique, physique, physico-chimique ou immunologique.

12. Procédé selon l'une quelconque des revendications précédentes, **caractérisé en ce que** le dosage des protéines est réalisé par mise en contact de tout ou partie de la solution d'extraction avec au moins un composé capable de réagir avec lesdites protéines et de manifester cette interaction selon un mode colorimétrique.

13. Kit utile pour caractériser l'état protéique de fibre(s) kératinique(s), comprenant au moins :
- une solution contenant de l'urée, de la thiourée ou un de leurs dérivés,
- au moins un réducteur autre que la thiourée, et
- au moins un composé utile à titre d'indicateur colorimétrique pour doser qualitativement et/ou quantitativement les protéines kératiniques extraites.

14. Kit selon la revendication 13, utile pour détecter au niveau des fibres kératiniques un antécédent de traitement par technologie alcaline de défrisage ou de lissage.

15. Kit selon la revendication 13 ou 14, **caractérisé en ce que** le réducteur est tel que défini en revendications 5 à 8.

16. Kit selon l'une quelconque des revendications 13 à 15, **caractérisé en ce que** la solution comprenant au moins l'urée et la thiourée et le réducteur sont conditionnés dans un même dispositif (20).

17. Kit selon l'une quelconque des revendications 13 à 15, **caractérisé en ce que** la solution comprenant l'urée et la thiourée et le réducteur sont conditionnés dans des récipients séparés (2,4).

18. Kit selon l'une quelconque des revendications 13 à 17, **caractérisé en ce qu'**il comprend en outre un récipient destiné à recevoir la solution d'urée et de thiourée, le réducteur et les fibres kératiniques.

19. Kit selon l'une quelconque des revendications 13 à 18, **caractérisé en ce que** l'indicateur colorimétrique est conditionné dans un récipient spécifique (5).

20. Kit selon l'une quelconque des revendications 13 à 19, **caractérisé en ce qu'**il comprend en outre une notice d'utilisation (6).

21. Kit selon l'une quelconque des revendications 13 à 20, **caractérisé en ce qu'**il comprend en outre un témoin colorimétrique et/ou un nuancier (7) permettant de doser qualitativement et/ou quantitativement les protéines extraites en fonction de l'intensité de la couleur obtenue à l'issue du test.

## Claims

1. Process for detecting, in keratinous fibre(s), a previous treatment by alkaline technology for hair straightening or smoothing comprising at least bringing the said keratinous fibre(s) into contact with an "extraction" solution comprising at least urea and thiourea or on of their derivatives, in a mixture with at least one reducing agent other than thiourea, under conditions which effective in dissolving keratin proteins, the qualitative and/or quantitative assaying of the proteins of the said extraction solution, the characterization of a previous alkaline treatment for hair straightening or smoothing by detecting a very small, or even zero, amount of proteins in dissolved form.

2. Process according to claim 1, **characterized in that** it. comprises the qualitative assaying of the proteins dissolved in the extraction solution.

3. Process according to claim 1 or claim 2, **characterized in that** the extraction solution comprises a mixture of urea an thiourea in a urea/thiourea ratio varying from 5/1 to 1/2, in particular from 4/1 to 2/1.

4. Process according to claim 3, **characterized in that** the said mixture is present in a proportion of 20% to 80% by weight, in particular of 30 to 70% by weight, with respect to the total weight of the said extraction solution.

5. Process according to any one of preceding claims, **characterized in that** the said solution comprises at least on reducing agent of thiol type.

6. according to claim 5, **characterized in that** the said reducing agent is chosen from thioglycolic acid or thiolactic acid and their ester and amide derivatives, in particular glyceryl monothioglycolate, cysteamine and its C₁-C₄ acylated derivatives, such as N-acetylcysteamine or N-propionylcysteamine, cysteine, N-acetylcysteine, thiomalic acid, pantetheine, 2,3-dimercaptosuccinic acid, sulphites or bisulphites of an alkali metal or alkaline earth metal, N-(mercaptoalkyl)-ω-hydroxyalkylamides, N-mono- or N,N-dialkyl-4-mercaptobutyramides, aminomercaptoalkylamides, derivatives of N-(mercaptoalkyl) succinamic acids and N-(mercaptoalkyl) succinamides, alkylaminomercaptoalkylamides, the azeotropic mixture of 2-hydroxypropyl thioglycolate and of 2-hydroxy-1-methylethyl thioglycolate, mercaptoalkylaminoamides, N-mercaptoalkylalkanediamides and formamidinesulphinic acid derivatives.

7. Process according to claim 6, **characterized in that** the said reducing agent is chosen from 2-mercaptoethanol, dithiothreitol (DTT), thioglycolic acid, one of their derivatives or of their mixtures.

8. Process according to any on of the preceding claims, **characterized in that** the said reducing agent is present in a proportion of 0.01 M to 2 M, in particular of 0.03 M to 0.5 M and more particularly of 0.04 M to 0.1 M in the extraction solution.

9. Process according to any one of the preceding claims, **characterized in that** the keratinous fibres and the extraction solution are brought into contact in a proportion of 0.2 to 100 mg, more particularly in a proportion of 1 to 50 g and in particular in a proportion of approximately 10 mg of keratinous fibres per ml of extraction solution.

10. Process according to any one of the preceding claims, **characterized in that** the keratinous fibres are immersed in the extraction solution and the combined product is heated to a temperature greater than 50°C, more particularly greater than 70°C and in particular greater than or equal to 100°C.

11. Process according to any one of the preceding claims, **characterized in that** the proteins are assayed by a chemical, physical, physicochemical or immunological assaying method.

12. Process according to any one of the preceding claims, **characterized in that** the proteins are assayed by bringing all or part of the extraction solution into contact with at least one compound capable of reacting with the said proteins and of displaying this interaction in a colorimetric form.

13. Kit of use in characterizing the protein state of keratinous fibre(s), the kit comprising at least:
• one solution comprising urea and thiourea, or one of their derivatives;
• at least one reducing agent other than thiourea;
and
• at least one compound of use as colorimetric indicator for qualitatively and/or quantitatively assaying the keratin proteins extracted.

14. Kit according to claim 13, of use in detecting, in keratinous fibres, a previous treatment by alkaline technology for hair straightening or smoothing.

15. Kit according to claim 13 or claim 14, **characterized in that** the reducing agent is as defined in claims 5 to 8.

16. Kit according to any one of claims 13 to 15, **characterized in that** the solution comprising at least urea an thiourea an the reducing agent are packaged in the same device (20).

17. Kit according to any one of claims 13 to 15, **characterized in that** the solution comprising urea and thiourea and the reducing agent are packaged in separate containers (2, 4).

18. Kit according to any one of claims 13 to 17, **characterized in that** it additionally comprises a container intended to receive the solution of urea and of thiourea, the reducing agent an the keratinous fibres.

19. Kit according to any one of claims 13 to 18, **characterized in that** the colorimetric indicator is packaged in a specific container (5).

20. Kit according to any one of claims 13 to 19, **characterized in that** it additionally comprises operating instructions (6).

21. Kit according to any one of claims 13 to 20, **characterized in that** it additionally comprises a colorimetric control and/or a color chart (7) which makes it possible to qualitatively and/or quantitatively assay the proteins extracted as a function of the intensity of the color obtained on conclusion of the test.

## Patentansprüche

1. Verfahren, um auf Höhe der Keratinfaser(n) einen Behandlungsvorgänger der Entkrausung oder Glättung durch alkalische Technologie zu erfassen, umfassend nündestens das In-Kontakt-Bringen der Keratinfaser(n) mit einer sogenwmten Extraktionslösung, die mindestens Harnstoff und Thioharnstoff oder eines ihrer Derivate in Mischung mit mindestens einem anderen Reduktionsmittel als Thiohamstoff umfasst, unter Bedingungen, die wirksam sind, um Keratinproteine zu solubilisieren, und die qualitative und/oder quantitative Bestimmung der Proteine der Extraktionslösung, und die Charakterisierung eines Vorgängers der alkalischen Behandlung der Entkrausung oder Glättung durch die Erfassung einer sehr reduzierten Menge, sogar Null, von Proteinen in solubilisierter Form.

2. Verfähren nach Anspruch 1, **dadurch gekennzeichnet, dass** es die qualitative Bestimmung der in der Extraktionslösung solubilisierten Proteine umfasst.

3. Verfahren nach Anspruch 1 oder 2, **dadurch gekennzeichnet, dass** die Extraktionslösung eine Mischung von Harnstoff und in einem Verhältnis Harnstoff/Thioharnstoff das von 5/1 bis 1/2, insbesondere von 4/1 bis 2/1 variiert.

4. Verfähren nach anspruch 3, **dadurch gekennzeichnet**, die Mischung in einem Anteil von 20 bis 80 Gew.-%, insbesondere von 30 bis 70 Gew.-%, bezogen auf das Gesamtgewicht der Extraktionslösung, vorliegt.

5. Verfahren nach der vorhergehenden Ansprüche, **dadurch gekennzeichnet, dass** die Lösung mindestens ein Reduktionsmittel vom Typ Thiol umfasst.

6. Verfahren nach anspruch 5, **dadurch gekennzeichnet**, das Reduktionsmittel ausgewählt ist aus Thioglycolsäure oder Thiomilchsäure und ihren Ester- und Amidderivaten, insbesondere Glycerinmonothioglycolat, Cysteamin und seinen C₁-C₄-acylierten Derivaten, wie N-Acetyleysteamin oder N-Propionylcysteamin, Cystein, N-Acetylcystein, Thiomalsäure, Panthetein, 2,3-Dimercaptosuccinsäure, den Sulfiten oder Bisulfiten eines Alkali- oder Erdalkalimetalls, den N-(Mercaptoalkyl)-□-hydroxyalkylamiden, den N-mono- oder N,N-Dialkylmercapto-4-butyramiden, den Aminomercaptoalkylamiden, den Derivaten der N-(Mereaptoalkyl)succinansäuren und der N-(Mereaptoalkyl)succinimide, den Alkylaminomercaptoalkylamiden, der azoltropen Mischung von 2-Hydroxypropylthioglycolat und (2-Hydroxy-1-methyl)ethylthioglycolat, den Mercaptoalkylaminoamiden, den N-Mercaptoalkylalcandiamiden und den Sulfinformamidinsäurederivaten.

7. Verfahren nach Anspruch 6, **dadurch gekennzeichnet, dass** das Reduktionsmittel aus 2-Mercaptoethanol, Dithiothreitol (DTT), Thioglycolsäure, einem ihrer Derivate oder ihrer Mischungen ausgewählt ist.

8. Verfahren nach einem der vorhergehenden Ansprüche, **dadurch gekennzeichnet, dass** das Reduktionsmittel in einer Menge von 0,01 bis 2 M, insbesondere 0,03 bis 0,5 M und vor allem von 0,04 bis 0,1 M in der Extraktionslösung vorliegt.

9. Verfahren nach einem der vorhergehenden Ansprüche, **dadurch gekennzeichnet, dass** die Keratinfasern und die Extraktionslösung in einer Menge von 0,2 bis 100 mg, insbesondere in einer Menge von 1 bis 50 mg und vor allem in einer Menge von etwa 10 mg Keratinfasern Extraktionslösung in Kontakt gebracht werden.

10. Verfahren nach einem der vorhergehenden Ansprüche, **dadurch gekennzeichnet, dass** die Keratinfasern in die Extrakfionslösung eingetaucht werden und das Ganze auf eine Temperatur über 50°C, insbesondere über 70°C und vor allem über oder gleich 100°C gebracht wird.

11. Verfahren nach einem der vorhergehenden Ansprüche, **dadurch gekennzeichnet, dass** die Bestimmung der Proteine mit Hilfe einer chemischen, physikalischen, physikalischchenüschen oder immunologischen Methode vorgenommen wird.

12. Verfahren nach einem der vorhergehenden Ansprüche, **dadurch gekennzeichnet, dass** die Bestünmung der Proteine durch In-Kontäkt-Bringen der ganzen Extraktionslösung oder eines Teils davon mit mindestens einer Verbindung vorgenommen wird, die in der Lage ist, mit diesen Proteinen zu reagieren und diese Interaktion auf eine kolorimetrische Weise zu manifestieren.

13. Set zur Kennzeichnung des Proteinzustands der Keratinfaser(n), umfassend mindestens:
- eine Lösung, die Harnstoff und Thioharnstoff oder eines ihrer Detivate enthält,
- ein anderes Reduktionsmittel als Thiohamstoff und
- mindestens eine Verbindung, die als kolorimetrischer indikator zur qualitativen und/oder quantitativen Bestimmung der extrahiehen Keratinproteine dient.

14. Set nach Anspruch 13, das dazu dient, auf Höhe der ¨Keratinfasern einen Behandlungsvorgänger der Entkrausung oder Glättung durch alkalische Technologie, zu erfassen.

15. Set nach Anspruch 13 oder 14, **dadurch gekennzeichnet, dass** das Reduktionsmittel so ist, wie in den Ansprüchen 5 bis 8 definiert ist.

16. Set nach einem der Ansprüche 13 bis 15, **dadurch gekennzeichnet, dass** die mindestens Harnstoff und Thioharnstoff umfassende Lösung und das Reduktionsmittel in einer gemeinsamen Vorrichtung (20) verpackt sind.

17. Set nach einem der Ansprüche 13 bis 15, dadurch gekennzeidnet, dass die Harnstoff und Thioharnstoff umfassende Lösung und das Reduktionsmittel in getrennten behältern (2, 4) verpackt sind.

18. Set nach einem der Ansprüche 13 bis 17, **dadurch gekennzeichnet, dass** es außerdem einen Behälter umfasst, der dazu bestimmt ist, die Harnstoff- und Thioharnstofflösung, das Reduktionsmittel und die Keratinfasern aufzunehmen.

19. Set nach einem der Ansprüche 13 bis 18, **dadurch gekennzeichnet**, der kolorimetrische Indikator in einem spezifischen Behälter (5) verpackt ist.

20. Set nach einem der Ansprüche 13 bis 19, **dadurch gekennzeichnet**, es außerdem eine Benutzungsanweisung (6) umfasst.

21. Set nach einem der Ansprüche 13 bis 20, **dadurch gekennzeichnet, dass** es außerdem einen kolorimetrischen Vergleich und/oder eine Farbskala (7) umfasst, der bzw. die es gestatten, die extrahierten Proteine in Abhängigkeit von der nach dem Test erhaltenen Intensität der Farbe qualitativ und/oder quantitativ zu bestimmen.
